Europäisches Patentamt

European Patent Office

Office européen des brevets

(11) Publication number : **0 546 796 A1**

# (12) EUROPEAN PATENT APPLICATION

(21) Application number : 92311163.7

(51) Int. Cl.$^5$ : **A61K 31/195**

(22) Date of filing : 08.12.92

(30) Priority : 12.12.91 JP 329135/91

(43) Date of publication of application :
16.06.93 Bulletin 93/24

(84) Designated Contracting States :
DE FR GB

(71) Applicant : **AJINOMOTO CO., INC.**
**15-1, Kyobashi 1-chome, Chuo-ku**
**Tokyo 104 (JP)**

(72) Inventor : **Sonaka, Ichiro, c/o Central Research**
**Lab.**
**Ajinomoto Co. Inc., No.1-1 Suzuki-cho,**
**Kawasaki-ku**
**Kawasaki-shi, Kanagawa-ken (JP)**

Inventor : **Umezawa, Tsutomu, c/o Central**
**Research Lab.**
**Ajinomoto Co. Inc., No.1-1 Suzuki-cho,**
**Kawasaki-ku**
**Kawasaki-shi, Kanagawa-ken (JP)**
Inventor : **Futami, Yuko, c/o Central Research**
**Lab.**
**Ajinomoto Co. Inc., No.1-1 Suzuki-cho,**
**Kawasaki-ku**
**Kawasaki-shi, Kanagawa-ken (JP)**
Inventor : **Kobayashi, Tetsuo, c/o Central**
**Research Lab.**
**Ajinomoto Co. Inc., No.1-1 Suzuki-cho,**
**Kawasaki-ku**
**Kawasaki-shi, Kanagawa-ken (JP)**
Inventor : **Maki, Toshio, c/o Central Research**
**Lab.**
**Ajinomoto Co. Inc., No.1-1 Suzuki-cho,**
**Kawasaki-ku**
**Kawasaki-shi, Kanagawa-ken (JP)**

(74) Representative : **Nicholls, Kathryn Margaret et**
**al**
**Mewburn Ellis, 2 Cursitor Street**
**London EC4A 1BQ (GB)**

(54) Treatment of Atherosclerosis.

(57)    L-arginine, its pharmaceutically acceptable salts, derivatives and analogs, are effective to reduce cholesterol ester accumulation in arterial walls and are therefore useful in the treatment and prevention of atherosclerosis.

EP 0 546 796 A1

EP 0 546 796 A1

The present invention relates to the treatment of atherosclerosis and to the manufacture of a pharmaceutical composition for use in such treatment.

Atherosclerosis is a type of arteriosclerosis and is a chronic disease of the arteries characterised by irregularly distributed lipid deposits in the intima of large and medium arteries. These deposits are associated with fibrosis and calcification. The causes of atherosclerosis are not well understood. However, the deposition of lipids is believed to be a prerequisite for the development of the arterial lesions which accompany the disease. Cholesterol esters constitute the major portion of the lipids which accumulate in atherosclerosis foci, and other components include free cholesterol, acylglycerols, phospholipids and related compounds.

Atherosclerosis is a major cause of cerebrocardiac vascular disorders such as brain infarction, heart infarction, and angina pectoris. Such cerebrocardiac vascular disorders account for around 40% of all adult deaths. Despite the key role of atherosclerosis in these disorders no fundamental method of treating or, in particular, of preventing it has yet been developed. In general, current treatments of atherosclerosis depend on the use of drug therapies intended to reduce hyperlipidaemia which is a significant risk factor in atherosclerosis. Clinical trials have confirmed that hyperlipidaemia treatment can lower the death rate resulting from coronary artery diseases (Lipid Research Clinics Program: J.A.M.A. <u>251</u>, 351-364 (1984)).

However, the relationship between arterial disease and hyperlipidaemia has not been completely elucidated so that there exists a desire in the art to discover a treatment for atherosclerosis which acts by a mechanism different from that of drug therapies for hyperlipidaemia. A particular problem arises because, according to one view, atherosclerosis develops in adolescence and progresses gradually from then on, although the onset of thrombotic diseases such as infarction, may not occur until middle or old-age. It is therefore desirable that an atherosclerosis treatment be available which may be administered over years, or even decades without risk of adverse side effects.

The present inventors have found that the amino acid L-arginine has the effect of suppressing the accumulation of cholesterol and cholesterol esters on the arterial wall and, thus, has an anti-atherosclerosis action.

According to the present invention, there is provided the use of a compound selected from L-arginine, pharmaceutically acceptable salts, derivatives and analogs of L-arginine, and mixtures thereof, in the manufacture of a medicament for the treatment or prevention of atherosclerosis in mammals, in particular in humans.

Also provided, according to a second aspect of the invention, is a pharmaceutical composition, for the treatment or prevention of atherosclerosis, comprising a compound selected from L-arginine, pharmaceutically acceptable salts, derivatives and analogs of L-arginine, and mixtures thereof; and a pharmaceutically acceptable excipient, diluent or carrier.

L-arginine is a semi-essential amino acid in mammals and is a well known, and widely available, chemical compound.

Pharmaceutically acceptable salts of L-arginine, which may be employed in the method and composition of the present invention, will be apparent to those in the art. They may be acid addition salts, or those formed with suitable bases. Illustrative acids which add to L-arginine to form pharmaceutically acceptable salts are, for example, inorganic acids such as hydrochloric acid, hydrobromic acid, sulfuric acid and phosphoric acid; and organic acids including acetic acid, lactic acid, citric acid, tartaric acid, maleic acid, fumaric acid and monomethylsulfuric acid. Typical pharmaceutically acceptable salts of L-arginine with bases are those with, eg. inorganic bases such as sodium, potassium or ammonia; and those with organic bases such as ethylenediamine, propylenediamine, ethanolamine, monoalkylethanolamines, dialkylethanolamines, diethanolamine or triethanolamine.

Instead of, or as well as, L-arginine or a pharmaceutically acceptable salt thereof, derivatives and/or analogs of L-arginine may be employed. These include compounds such as L-arginine "pro-drugs" which are converted to L-arginine in the body, for example by enzymatic conversion. Esters of L-arginine and small peptides including L-arginine would fall into this category. Other possibilities will be apparent to those of skill in the art.

The L-arginine, salt, derivative and/or analog may be administered by any suitable route; in particular orally, or intravenously; by infusion, or by injection. The L-arginine active ingredient may be formulated together with an excipient, diluent or carrier appropriate to the chosen route of administration. The pharmaceutical composition so formed may be in the form of a powder, granules, tablet, capsule, suspension or solution. Where the composition is in the form of an aqueous solution it may be appropriate that it is any or all of: buffered; sterile; and isotonic.

The L-arginine, salt, analog and/or derivative may be administered in conjunction with other active ingredients but, if so, it will generally be present in a larger quantity by weight than any other active ingredient and, in particular in a larger amount by weight than all other active ingredients together. When the L-arginine, salt, derivative or analog is included in a pharmaceutical composition together with a pharmaceutically acceptable excipient, diluent or carrier, it may, nevertheless, constitute the major component of the composition, ie. be present at more than 50% by weight. The pharmaceutical composition of the present invention may consist

2

essentially of a compound selected from L-arginine, its pharmaceutically acceptable salts, analogs and derivatives, and mixtures thereof.

The mode of administration and the amount of compound administered will vary from patient to patient depending on the age and general condition of the patient as well as the state of advancement of the patient's atherosclerosis. Ultimate choice of dose will be at the discretion of the clinician. However, a suitable daily dose of L-arginine, salt, derivative and/or analog for an adult (who may weigh around 65kg) is likely to be in the range about $6 \times 10^{-4}$ to about 0.6 moles (0.1 to 100g of L-arginine), preferably about $6 \times 10^{-3}$ to about 0.3 moles (1 to 50g of L-arginine), and particularly about $6 \times 10^{-2}$ to about 0.2 moles (10 to 30g of L-arginine). The L-arginine, salt, derivative and/or analog may be arranged in a dosage form such that, by appropriate dosing daily doses in the ranges stated above may be achieved.

Embodiments of the present invention are illustrated below by way of example only.

Examples

Example 1

Eight-week old New Zealand white rabbits underwent 8 weeks of restricted feeding with 100g daily of an experimental diet (commercially available RC4 for the control group, including 1% cholesterol for the "1% cholesterol-medicated group", and 1% cholesterol + 5% L-arginine for the "5% L-arginine-medicated group"). The number of animals was 5 per group. After 8 weeks a blood sample was collected via the auricular artery, and the total cholesterol content of the plasma was determined by the cholesterol oxidase method. The animals were subjected to abdominal section under pentobarbital anaesthesia, and then bled to death. The abdominal aorta was extirpated, and its total cholesterol and cholesterol ester contents were determined by the cholesterol oxide method. The results are laid out in Table 1.

Table 1 lists the total cholesterol content of each of plasma and abdominal aorta, and the cholesterol esters content of the latter. The cholesterol in the plasma increased 20 to 30 times due to the addition of cholesterol to the diet, and this increase was not suppressed by the addition of L-arginine. However, a significant increase in the total cholesterol content and in the cholesterol ester content of the abdominal aorta which was caused by the addition of cholesterol was observed to be remarkably lowered by the addition of L-arginine.

Table 1

Cholesterol contents of plasma and abdominal aorta

| | Control Group | 1% cholesterol medicated group | 1% cholesterol + 5% arginine - medicated group |
|---|---|---|---|
| Cholesterol in plasma (mg/dl) | 57±7 | 1251±95* | 1651±70*# |
| Total cholesterol in abdominal aorta (mg/g) | 4.4±0.9 | 20.2±3.6* | 12.3±1.4*# |
| Cholesterol esters in abdominal aorta (mg/g) | 0.9±0.4 | 11.9±2.9* | 6.1±1.1* |

*: $p < 0.05$ vs control group;

#: $p < 0.05$ vs 1% cholesterol-medicated group

Example 2

Male New Zealand white rabbits at 8 weeks of age were fed one of the following diets: normal rabbit chow;

normal rabbit chow including 1% cholesterol; normal rabbit chow including 1% cholesterol supplemented with 1% L-arginine; normal rabbit chow including 1% cholesterol supplemented with 3% L-arginine; or normal rabbit chow including 1% cholesterol supplemented with 5% L-arginine. After 4 weeks, the thoracic aorta was removed and the cholesterol content of the ascending aorta and arch was determined by the cholesterol oxidase method.

The results are shown in Table 2. The total cholesterol content of aortic wall in rabbits fed a diet including 1% cholesterol and no arginine was 4 fold higher than normal rabbits. By contrast, arginine (1% to 5%) caused a dose-related reduction in the total cholesterol increase. Accumulation of esterified cholesterol in aortic wall was observed in cholesterol fed rabbits, however, the accumulation was suppressed significantly in rabbits fed the 3% and 5% arginine supplemented diets.

Table 2 Effect of L-arginine on cholesterol content of ascending aorta and arch in hypercholesterolemic rabbits.

|  | Total cholesterol | Esterified cholesterol |
|---|---|---|
|  | mg/g dry wt | |
| Normal(6) | $5.81 \pm 0.91^c$ | N.D. |
| Chol(5) | $21.25 \pm 6.52^a$ | $10.86 \pm 4.24^a$ |
| Chol+1%Arg(5) | $14.50 \pm 2.91^b$ | $6.75 \pm 1.87^{ab}$ |
| Chol+3%Arg(5) | $12.27 \pm 3.89^b$ | $5.32 \pm 2.33^b$ |
| Chol+5%Arg(5) | $10.32 \pm 1.55^{bc}$ | $4.25 \pm 1.31^b$ |

Values are mean$\pm$SD. The number of animals is given in parentheses. N.D. means not detected. Values within a given column with the same superscript letter are significantly different between groups at $p<0.05$ assessed by Bonferroni's multi comparison test.

## Claims

1. Use of a compound selected from L-arginine, pharmaceutically acceptable salts, derivatives and analogs of L-arginine and mixtures thereof in the manufacture of a medicament for the treatment or prevention of atherosclerosis in a mammal.

2. A use according to claim 1 wherein the mammal is a human.

3. A use according to claim 1 or claim 2 wherein the compound is arranged for administration orally or by injection.

4. A use according to any one of the preceding claims wherein the compound is arranged for administration in a daily dose of from about $6\times10^{-4}$ to about 0.6 moles.

5. A use according to claim 4 wherein the compound is arranged for administration in a daily dose of from about $6\times10^{-3}$ to about 0.3 moles.

6. A use according to claim 5 wherein the compound is arranged for administration in a daily dose of from about $6\times10^{-2}$ to about 0.2 moles.

7. A pharmaceutical composition for the treatment or prevention of atherosclerosis comprising: an effective amount of a compound selected from L-arginine, pharmaceutically acceptable salts, derivatives and analogs of L-arginine, and mixtures thereof; and a pharmaceutically acceptable excipient, diluent or carrier.

8. A pharmaceutical composition according to claim 7 wherein said compound is the principal component of the composition.

**European Patent Office**

## EUROPEAN SEARCH REPORT

Application Number

EP 92 31 1163

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CIRCULATION<br>vol. 83, no. 3, 1991,<br>pages 1118 - 1120<br>G.B.RUBANYI 'Reversal of hypercholesterolemia-induced endothelial dysfunction by L-arginine'<br>* the whole document * | 1-8 | A61K31/195 |
| X | CIRCULATION<br>vol. 83, no. 3, 1991,<br>pages 1057 - 1062<br>J.P.COOKE ET AL 'Arginine restores cholinergic relaxation of hypercholesterolemic rabbit thoracic aorta'<br>* the whole document * | 1-8 | |
| X | GEN PHARMACOL.<br>vol. 21, no. 5, 1990,<br>pages 575 - 587<br>J.MARÍN ET AL 'Role of endothelium-formed nitric oxide on vascular responses.'<br>*summary, p. 583* | 1-8 | **TECHNICAL FIELDS SEARCHED (Int. Cl.5)**<br><br>A61K |
| X | ATHEROSCLEROSIS<br>vol. 43, no. 2-3, 1982,<br>pages 381 - 391<br>M.B.KATAN ET AL 'Reduction of casein-induced hypercholesterolaemia and atherosclerosis in rabbits and rats by dietary glycine, arginine and alanine.'<br>* the whole document * | 1-7 | |

The present search report has been drawn up for all claims

| Place of search | Date of completion of the search | Examiner |
|---|---|---|
| THE HAGUE | 22 MARCH 1993 | KLAVER T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)